# EUROPEAN PATENT APPLICATION

(11) **EP 2 481 447 A1**
(43) Date of publication of application: **01.08.2012**
(21) Application number: 11169900.5
(22) Date of filing: 14.06.2011
(51) Int. Cl.: A61N 7/02

(54) **High intensity focused ultrasonic medical instrument with dual transducers**

(30) Priority: 01.02.2011 KR 20110010143
(71) Applicant: Hironic Co., Ltd., Joongwon-gu Sungnam-si 462-120 (KR)
(72) Inventor: Lee, Jin U, Guri-si Gyeonggi-do (KR)
(74) Representative: Tersteegen, Felix

(57) **Abstract**

The present invention relates to a high intensity focused ultrasonic (HIFU) medical instrument with dual treatment transducers located around an imaging transducer. The treatment transducers are contained within an exchangeable cartridge. A graphical user interface on a touch-screen allows selection of icons that correspond to different facial regions to be treated. Selection of parameters adapted for a facelift of the selected region is thus possible. The emitted high intensity focused ultrasound is suitable to deliver ultrasonic heat to the superficial musculo-aponeurotic system (SMAS) and the subcutaneous tissue region and thereby perform a facelift (cosmetic surgical operation to remove unwanted wrinkles by tightening the skin of the face) and collagen remodelling effect.

## Description

### TECHNICAL FIELD

The present invention relates to a high intensity focused ultrasonic (HIFU) medical instrument with dual transducers, and more particularly, to a high intensity focused ultrasonic (HIFU) medical instrument with dual transducers which includes dual transducers which emitting high intensity focused ultrasound and is the surgical instrument without the invasive surgical incision in the patient's face, and deliver ultrasonic heat to the SMAS and the subcutaneous tissue region by using the ultrasound and thereby performing the facelift (acosmetic surgical operation to remove unwanted wrinkles by tightening the skin of the face) and collagen remodeling effect.

### BACKGROUND ART

Ultrasound is a wave with frequency of over 20KHz and it passes through water and is used in the medical areas of the ultrasonic diagnostic instrument, ultrasonic treatment device and so on.

The main usefulness of a ultrasound in the medical field is a ultrasonic image equipment which uses the ultrasonic properties such as the ultrasonic transmission and reflection. The ultrasound penetrates the organs of a human body and the medical equipment acquires sectional images within the human body by visualizing the reflected time and degree of intensity of the ultrasound.

Besides, the ultrasound has the following effects.
(1) Mechanical effect of the ultrasound
   The ultrasonic vibration is directly delivered to the surgical knife and it is used for the ultrasonic cutting and coagulation.
(2) Cavitational effect of the ultrasound
   When the sound pressure of the ultrasound passes through a fluid, tiny bubbles happen. The tiny bubbles burst off after expansion and breakdown and the shockwave occurs as a result of its high pressure and strong shearing force is accompanied.
(3) Thermal effect of Ultrasound
   When the ultrasound is transmitted into a tissue its energy is turned into heat. The ultrasound with enough energy may increase the heat rapidly.

The ultrasonic surgical device is a medical instrument using the thermal effect of the ultrasound. In particular, the high intensity focused ultrasonic surgical unit (HIFU) is a medical instrument of which the ultrasonic transducer emits ultrasound to a focused point in a skin tissue and cures the cancers such as a liver cancer, a uterine cancer, breast cancer and so forth by the ultrasonic heat of the focused point. It does not result in a wound, a side effect or a pain and it increases the quality of life for the patients.

Compared to the conventional invasive surgery, according to the HIFU since the external energy (ultrasound) is emitted into the human body it does not need to cut a skin tissue and the curing method is simple. After the surgical procedure recovery time is likely to be reduced so that the demand for the HIFU is on the increase.

Recently, the HIFU is applied to the facelift procedure for the increase of quality of life. Combination of the relatively low level of strength of the ultrasonic energy (less than 100W) and the focused ultrasound with minute focused point does have a good effect on the improvement of a skin tissue so that the HIFU emerges as an alternative to the invasive surgical method.

The human tissue structure comprises epidermal layer, dermal layer, subcutaneous layer, muscle and bone in order. The dermal layer mainly comprises collagen which is well known protein and maintains skin elasticity.

The SMAS (Superficial Musculo-Aponeurotic System) layer is the part of the muscle which is pulled tight when the facelift is performed. The ultrasound of HIFU does not give an effect on an epidermal layer but induces coagulation function in the SMAS layer. And it transmit the heat of the ultrasound to the dermal layer so that it may reproduce collagen for smoothing out wrinkles and increasing skin elasticity in accordance with the medical reports overseas.

### DISCLOSURE OF THE INVENTION

### Technical Problem

There is a need for combined system of the ultrasonic transducer for cosmetic surgical purpose and its monitoring device. The ultrasonic transducers are in concave shape and an image probe can acquire the ultrasonic image in real time by emitting the ultrasound to the target regions to be treated within a face. A dermatologist can monitor the target region in the patient's skin tissue by the real time ultrasonic image probe during the cosmetic procedure of facelift so that the safety and accuracy of the cosmetic procedure can be increased.

### Technical Solution to Problem

To accomplish the above-mentioned object, according to an aspect of the invention, there is provided a high intensity focused ultrasonic (HIFU) medical instrument with dual transducers comprising: dual transducers 52 which emitting ultrasound and installed within a cartridge 50; and wherein said dual transducers 52 emitting said ultrasound to a same focus 60, an image probe 42 which being placed in the middle of said dual transducers 52; wherein said image probe 42 obtaining an ultrasonic internal image of a skin tissue by emitting said ultrasound. In one embodiment, said cartridge 50 being removable from said image probe 42 separately.

The high intensity focused ultrasonic (HIFU) medical instrument with dual transducers providing images of figuration having optimized parameters on different parts of a face of a patient for facelift and a dermatologist click on said image of figuration, wherein said image of figuration classified by target regions on said patient's face for said facelift. The high intensity focused ultrasonic (HIFU) medical instrument with dual transducers further comprising: an image monitoring display panel 10 which displaying ultrasonic image of said patient's skin tissue within a face which being obtained by said image probe 42; and, a ultrasonic controlling display panel 20 which being a touch screen on which strength of ultrasound being controlled.

The high intensity focused ultrasonic (HIFU) medical instrument with dual transducers further comprising: a main body; and wherein said main body comprising: a system controller 12 which controlling each component of said medical instrument; a ultrasound emitting module 53 which making said dual transducers 52 generating ultrasound for cosmetic procedure; an image monitoring display panel 10 which displaying an ultrasonic image obtained from said image probe 42; and a ultrasonic controlling display panel 20 which made of a touch screen and being an handling equipment for the dermatologist; a cartridge 50 within which said dual transducers 52 being installed; and a hand wand 30 which insetting with said removable cartridge 50 and used by a dermatologist.

### Advantageous Effect

According to the above-mentioned configurations of the high intensity focused ultrasonic medical instrument having dual transducers, the present invention has two transducers which are emitting ultrasound at the same focus for the cosmetic procedure so that the intensity of ultrasound from each transducer can be reduced, thereby preventing blood vessels or nerves of a patient's skin tissue within the face from being damaged.

And the dermatologist can detect the precise location of target region of the patient's skin tissue for the facelift with the hand wand in which the image probe is installed.

And the present invention has dual controlling systems: one is the dual-transducer system for lower ultrasonic intensity from each transducer and the other is the image probe system for detecting precise target region to be treated for the facelift.

The present invention also has a user friendly interface with each image with different setting parameter. The each image with the different setting parameter contains different strength of ultrasound and depth value which being preset for different parts of cosmetic procedure. The dermatologist does not need to putting in exact setting value for the facelift.

Since the dual transducers are installed within the cartridge, the ultrasonic intensity of each transducer may be reduced thereby increasing the lifetime of each transducer. In the end, the replacement cycle of the cartridge may lengthen.

The present invention with the above described advantageous effects is an high end medical instrument which have effects of smoothing out wrinkles and collagen remodeling.

### BRIEF DESCRIPTION OF THE DRAWINGS

Fig. 1 is a perspective view illustrating the high intensity focused ultrasonic medical instrument having dual transducers according to an embodiment of the invention.
Fig. 2 is an exploded perspective view illustrating a hand wand and a cartridge according to the embodiment of the invention.
Fig. 3 is a block diagram illustrating the composition of the high intensity focused ultrasonic medical instrument having dual transducers according to an embodiment of the invention.
Fig. 4 shows the way of emitting the ultrasound by the high intensity focused ultrasonic medical instrument having dual transducers according to an embodiment of the invention compared to that of the prior art.
Fig. 5 is a cross sectional view illustrating the cosmetic procedure by using the hand wand of the high intensity focused ultrasonic medical instrument having dual transducers according to an embodiment of the invention.
Fig. 6 is a front view of the monitor illustrating a system which controls an image and an ultrasonic focusing of the high intensity focused ultrasonic medical instrument having the dual transducers according to the present invention.
Fig. 7 is an exemplary view illustrating user friendly interface having each optimized parameter provided in the high intensity focused ultrasonic medical instrument according to the present invention.

### Description of Reference Numerals

- 10:: image monitoring display panel
- 12:: system controller
- 20:: ultrasonic controlling display panel
- 30:: hand wand
- 42:: image probe
- 43:: image monitoring system
- 50:: cartridge
- 52:: transducer
- 53:: ultrasound emitting module
- 54:: water
- 60:: focus
- 62:: patient's skin tissue

### DESCRIPTION OF EXEMPLARY EMBODIMENTS

The configuration and operation of the high intensity focused ultrasonic medical instrument having dual transducers according to an embodiment of the invention will be described in detail with reference to the accompanying drawings.

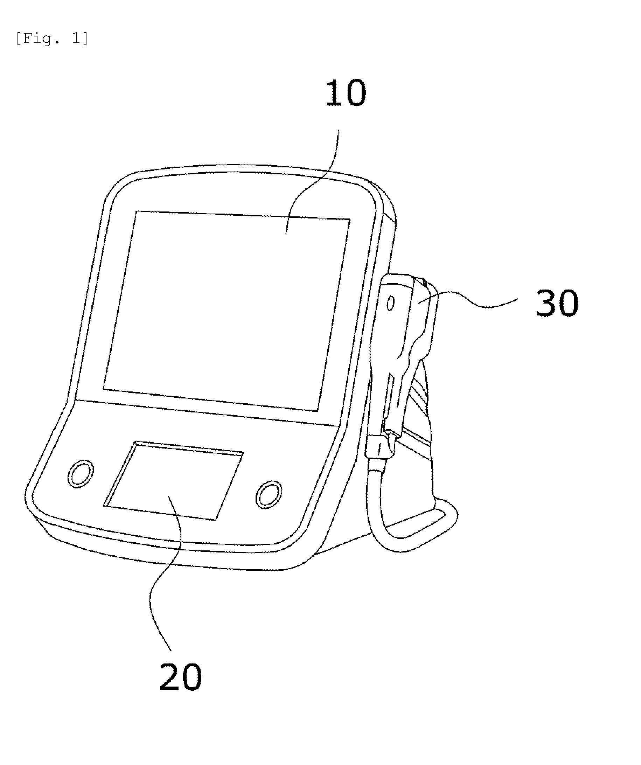

Fig. 1 is a perspective view illustrating the high intensity focused ultrasonic medical instrument having dual transducers according to an embodiment of the present invention.

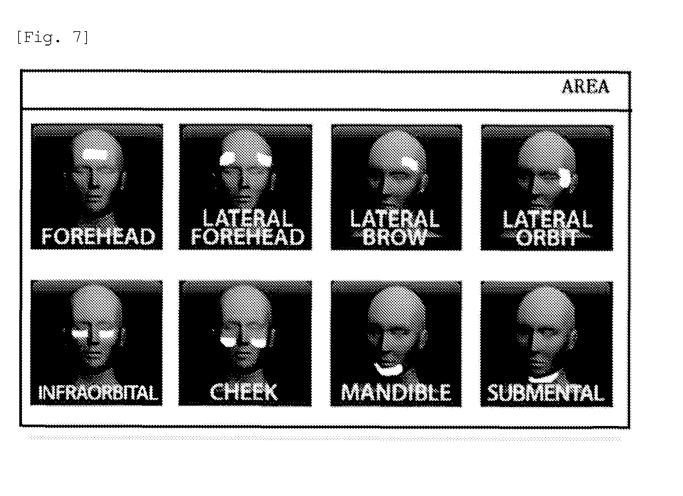

As shown in Fig.1, the high intensity focused ultrasonic medical instrument having dual transducers according to an embodiment of the present invention includes a displaying device having two touch screen LCD panels and a hand wand 30. The displaying device includes an image monitoring display panel 10 and a ultrasonic controlling display panel 20. The image monitoring display panel 10 on its upper part shows an image of a region of interest inside a patient's tissue and the ultrasonic controlling display panel 20 on its lower part shows the intensity of the ultrasound of the dual transducers, the parameterized figurations (referring to Fig. 7) and so forth.

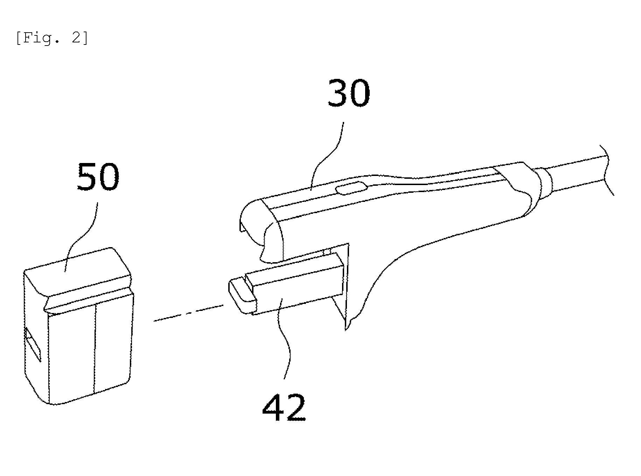

Fig. 2 is an exploded perspective view illustrating a hand wand and a cartridge according to the embodiment of the invention.

As shown in Fig. 2, a detachable cartridge 50 may be mounted on the front part of the hand wand 30 with an image probe 42 inserted into the detachable cartridge 50.

The dual transducers 52 are installed in the cartridge 50 according to the present invention and the each transducer 52 is placed on the left and right side of the inserting part of the image probe 42.

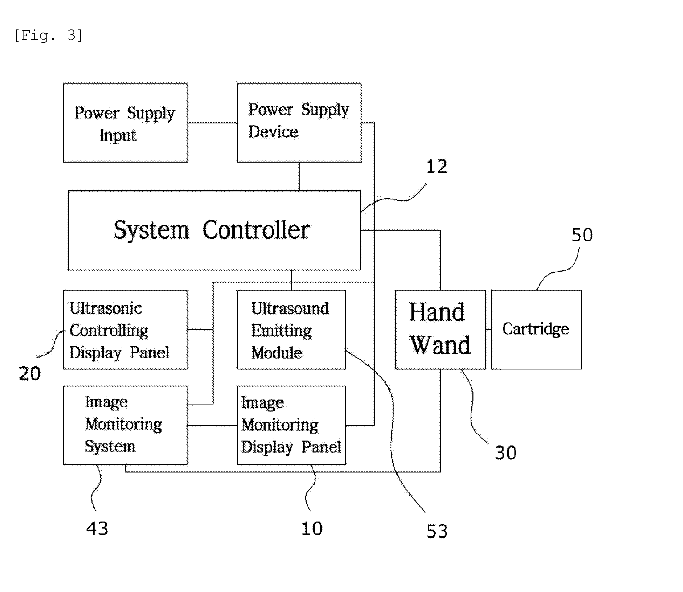

Fig. 3 is a block diagram illustrating the composition of the high intensity focused ultrasonic medical instrument having dual transducers according to an embodiment of the present invention.

As shown in Fig. 3, the high intensity focused ultrasonic medical instrument having dual transducers according to an embodiment of the present invention includes the main body, the cartridge 50 and the hand wand 30.

The main body comprises an ultrasound emitting module 53 for cosmetic treatment, a system controller 12, the image monitoring display panel 10 and the ultrasonic controlling display panel 20 which is an operating method on the touch screen panel for the dermatologist's convenience. The cartridge 50 has two high intensity focused ultrasonic transducers 52 installed in it. The hand wand 30 is coupled with the cartridge 50 and used for the cosmetic treatment according to the present invention.

The high intensity focused ultrasonic medical instrument having dual transducers according to an embodiment of the invention is in operation after applying rated voltage to its main body.

When the surface of the ultrasonic transducer 52 which being in round and concave shape generates ultrasound, the ultrasound is getting focused and starts to generate a focus having high intensity energy on a particular location. When the ultrasound is emitted into the patient's skin tissue 62, the heat starts to be generated at the point of the focus 60 because of the heat effect of the ultrasound and with this heat the medical treatment may be performed at the targeted area of the skin tissue 62.

The principle of the operation of the high intensity focused ultrasonic medical instrument having dual transducers is as follows: the electric power is applied from the power supply device to the system controller 12, the ultrasonic controlling display panel 20 which is an operation panel, an imaging monitoring system 43 which is sensing and imaging the image of the targeted area of the skin tissue 62, the ultrasound emitting module 53 which is making the transducer 52 produce the high intensity ultrasound and the image monitoring display panel 10 which displays ultrasonic images.

When the system controller 12 starts to work, main operational menu is displayed on the ultrasonic controlling display panel 20. Setting the value for the cosmetic treatment may be performed on it. And the image probe 42 and the image monitoring display panel 10 will be turned on and ready to display an image of the targeted area of the patient's skin tissue 62.

When the setting value is finished on the ultrasonic controlling display panel, the value to be set is transmitted to the system controller 12. The system controlling 12 orders the ultrasound emitting module 53 to generate the ultrasound according to the set value. The high intensity focused ultrasound may be emitted through the hand wand 30 with the cartridge 50 mounted on.

The hand wand 30 includes the image probe 42 so that the real time ultrasonic image of the patient's skin tissue may be observed.

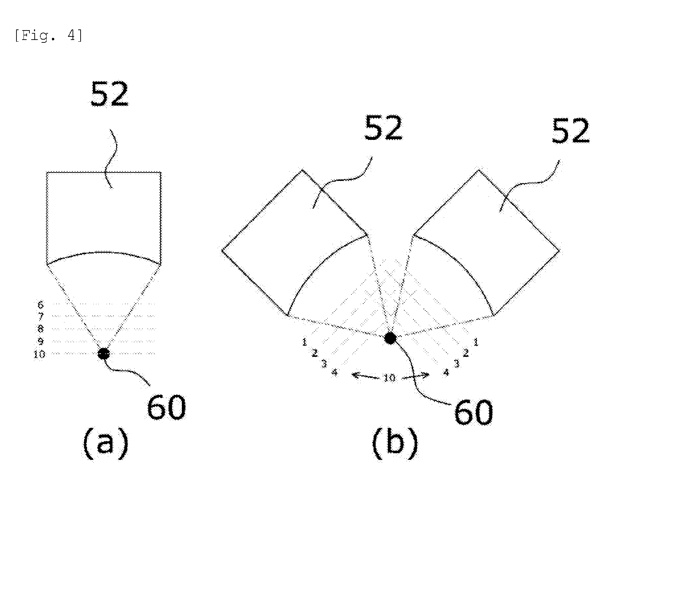

Fig. 4 shows the way of emitting the ultrasound by the high intensity focused ultrasonic medical instrument having dual transducers according to an embodiment of the invention compared to that of the prior art.

As shown in Fig. 4(a), conventionally, one transducer 52 has been used for the cosmetic treatment which is using the ultrasound. The target region of the skin for the dermatological cosmetic treatment is primarily a face and the cosmetic treatment is using the transducer 52 which is emitting the high intensity focused ultrasound. Conventionally, since the cosmetic procedure was carried out with one transducer 52, the strength of the ultrasound emitted by one transducer 52 has been very high for the cosmetic wrinkle treatment.

Since the target region of the skin for the cosmetic procedure is a face, wrong focus to be treated by a dermatologist may lead to damage to blood vessel or nerves within the patient's face. Especially, since only one transducer 52 has been used the strength of the ultrasound to be pointed at the focus 60 is very high, '10' for example. In case the target region of the skin to be treated is out of the focus 60, the damage may happen to blood vessel or nerves of other area of the focus 60 to be set beneath the surface of the patient's face.

The number 6, 7, 8, 9 and 10 on the figure 4 are exemplary strengths of the ultrasound at each location.

Referring to Fig. 4(b), the dual transducers 52 are installed within the cartridge 50 according to the high intensity focused ultrasonic medical instrument and the dual transducers 52 are in position where the dual transducers 52 are emitting the ultrasound to the same focus 60.

According to the embodiment as shown in Fig 4(b), the sum of strength of the ultrasound emitted from the dual transducers 52 is '10' as in Fig. 4(a), but the each strength of the ultrasound emitted from the each transducer 52 are 1, 2, 3, 4, 5 while the strength of the ultrasound of Fig 4(a) are 6, 7, 8, 9, 10 at the same each position.

Consequently, the each ultrasonic strength from the each transducer 52 as in Fig. 4(b) is value subtracting 5 from each value as in Fig. 4(a) at the each location. Each value of the each transducer 52 is 1 (6-5), 2 (7-5), 3 (8-5), 4(9-5), 5(10-5). With each reduced value of the ultrasonic strength of the transducer 52, the blood vessel and nerves above the focus 60 region of the skin to be treated are less likely to be damaged during the cosmetic procedure. Besides, even if it is out of the focus 60 it is unlikely to damage the blood vessel and nerves within the face.

The present invention uses two transducers 52 as a dual module and each transducer 52 does not need to emit high intensified ultrasound so that each transducer 52 may be more durable. As a result, the life cycle of the cartridge 50 with the dual transducers 52 installed in may be extended and it may result in the improvement of economical efficiency.

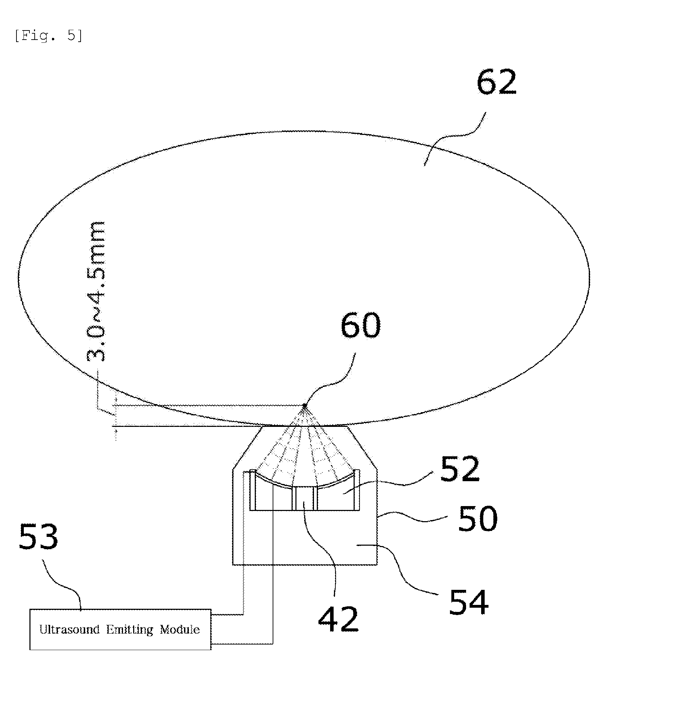

Fig. 5 is a cross sectional view illustrating the cosmetic procedure by using the hand wand of the high intensity focused ultrasonic medical instrument having dual transducers according to an embodiment of the invention.

Referring to Fig. 5, a patient's skin tissue 62 within the face is closely adhered to the cartridge 50 of the hand wand 30 according to the present invention. The ultrasound emitting module 53 of the main body originates the ultrasound and the ultrasound will be emitted to the focus 60 within the patient's skin tissue 62 which is the target region to be treated for the cosmetic procedure. The cartridge 50 is filled with water 54.

According to the present invention, the image probe 42 is installed in the center with the each transducer 52 placed in left and right so that the skin tissue around the focus 60 to which the ultrasound is to be emitted may be imaged by the image probe 42. The thermal coagulation of the skin tissue around the focus 60 may be monitored while the ultrasound is being emitted.

In accordance to the cross sectional view of the high intensity focused ultrasonic medical instrument according to the present invention, the ultrasound emitted from the each transducer 52 which is in concave shape is being focused on the center point of the transducer 52.

The dual transducers 52 are installed within the cartridge which is the air tight case and the water 54 is filled in the internal part of the cartridge 50. The water 54 within the cartridge 50 serves as a medium for transferring the ultrasound from the each transducer 52 to the patient's skin tissue 62 for the cosmetic procedure and also cools down the heat generated from the each transducer 52. And the dual transducers 52 work with the image probe 42 to obtain the cross sectional image of patient's skin tissue 62.

Since the dermatologist can obtain the ultrasonic image of the patient's skin tissue 62 by the image probe 42 so that he can identify the correct target region to be treated in real time. He can find out the correct focus 60 to be treated in the patient's skin tissue 62 and perform the accurate cosmetic-procedure such as a facelift by emitting the ultrasound.

As described above, the present invention can induce the accurate thermal-coagulation within the patient's skin tissue 62 and increase the safety for the facelift.

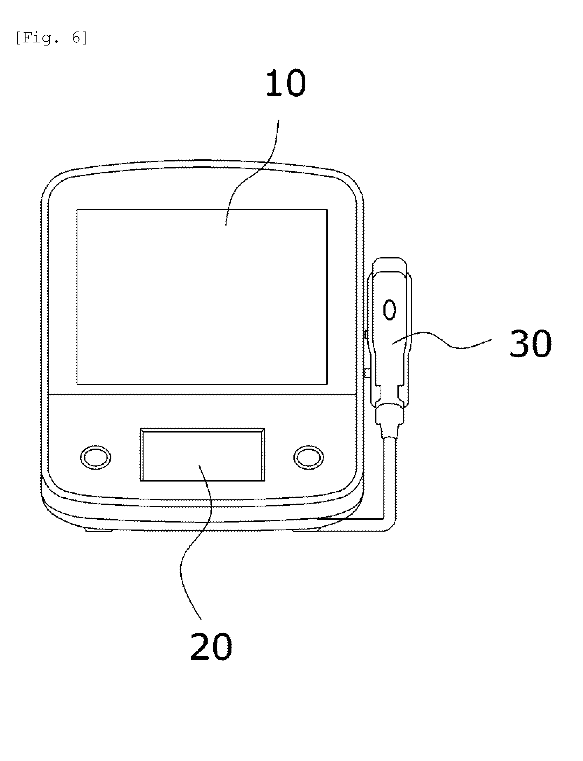

Fig. 6 is a front view of the monitor illustrating a system which separately controls an image and an ultrasonic focusing of the high intensity focused ultrasonic medical instrument having the dual transducers according to the present invention.

Referring to Fig. 6, the dermatologist may obtain the ultrasonic image of the target region of the patient's skin tissue which is sensed by the image probe 42 on the image monitoring display panel 10 of the main body. Through the image he can check if the focus 60 of the dual transducers 52 points at the correct target region for the cosmetic-procedure. Additionally, he can control the strength of the ultrasound emitted from the dual transducers 52 on the ultrasonic controlling display panel 20.

According to the above composition of the present invention, the precise control and response for the facelift can be guaranteed.

Fig. 7 is an exemplary view illustrating a user friendly interface having each optimized parameter provided in the high intensity focused ultrasonic medical instrument according to the present invention.

Referring to the Fig. 7, the present invention may provide the dermatologist with a user friendly interface to provide convenient functions by extracting information such as thickness of a skin tissue, thickness of a bone, distribution of nerves, distribution of blood vessels, etc.

The user friendly interface program with figurations of front part of a brow, side of the brow, a cheekbone, jaw and so forth in Fig. 7 as an each example is installed in the system controller 12.

When the dermatologist determines the strength of ultrasound according to a target region to be treated for the cosmetic procedure, he may just touch the particular image of a figure on the ultrasonic controlling display panel 20 for the cosmetic procedure to the target region to be treated without putting in a value of strength of the ultrasound to be emitted and a particular location of the focus 60 in the patient's skin tissue.

Each image of figure which displayed on the ultrasonic controlling display panel 20 may contain precise value of the ultrasonic strength to be emitted to the target region and the location of the focus 60 to be emitted in the patient skin tissue 62.

While specific embodiments of the invention have been described, it will be obvious to those skilled in the art that the invention may be modified in various forms without departing from the scope of the invention and the modifications belongs to the scope of the appended claims.

## Claims

1. A high intensity focused ultrasonic (HIFU) medical instrument with dual transducers comprising:
dual transducers 52 which emitting ultrasound and
installed within a cartridge 50; and
wherein said dual transducers 52 emitting said ultrasound to a same focus 60,
an image probe 42 which being placed in the middle of said dual transducers 52;
wherein said image probe 42 obtaining an ultrasonic internal image of a skin tissue by emitting said ultrasound.

2. The high intensity focused ultrasonic (HIFU) medical instrument with dual transducers according to claim 1, wherein said cartridge 50 being removable from said image probe 42 separately.

3. The high intensity focused ultrasonic (HIFU) medical instrument with dual transducers according to claim 1, wherein said the high intensity focused ultrasonic (HIFU) medical instrument with dual transducers providing images of figuration having optimized parameters on different parts of a face of a patient for facelift and a dermatologist click on said image of figuration,
wherein said image of figuration classified by target regions on said patient's face for said facelift.

4. The high intensity focused ultrasonic (HIFU) medical instrument with dual transducers according to claim 1, said the high intensity focused ultrasonic (HIFU) medical instrument with dual transducers further comprising:
an image monitoring display panel 10 which displaying ultrasonic image of said patient's skin tissue within a face which being obtained by said image probe 42; and,
a ultrasonic controlling display panel 20 which being a touch screen on which strength of ultrasound being controlled.

5. The high intensity focused ultrasonic (HIFU) medical instrument with dual transducers according to claim 1, said high intensity focused ultrasonic (HIFU) medical instrument with dual transducers further comprising:
a main body; and
wherein said main body comprising:
a system controller 12 which controlling each component of said medical instrument;
a ultrasound emitting module 53 which making said dual transducers 52 generating ultrasound for cosmetic procedure;
an image monitoring display panel 10 which displaying an ultrasonic image obtained from said image probe 42; and
a ultrasonic controlling display panel 20 which made of a touch screen and being an handling equipment for the dermatologist;
a cartridge 50 within which said dual transducers 52 being installed; and
a hand wand 30 which insetting with said removable cartridge 50 and used by a dermatologist.
